# EUROPEAN PATENT SPECIFICATION

(11) **EP 2 278 329 B1**
(45) Date of publication and mention of the grant of the patent: **30.04.2014**
(21) Application number: 10170112.6
(22) Date of filing: 20.07.2010
(51) Int. Cl.: A61N 1/02, A61N 1/372, G06F 19/00, G01N 33/487

(54) **Active interface device**
Aktive Schnittstellenvorrichtung
Dispositif d'interface active

(30) Priority: 20.07.2009 US 227001 P; 29.01.2010 EP 10152166
(43) Date of publication of application: 26.01.2011
(73) Proprietor: IMEC, 3001 Leuven (BE); Katholieke Universiteit Leuven, K.U.L. Leuven R&D, 3000 Leuven (BE)
(72) Inventor: Aslam, Junaid, Tehsil and District Gujrat (PK); Merken, Patrick, B-3890, Montenaken (BE); Van Hoof, Chris, B-3001, Leuven (BE)
(74) Representative: Pronovem

(56) References cited:
- WO-A2-2007/058950
- US-A- 4 736 751
- US-A1- 2002 192 637
- US-A1- 2004 106 101
- US-A1- 2006 089 112
- US-A1- 2007 129 770

## Description

### Field of the invention

The present invention relates to the field of interface devices comprising a plurality of transducers or sensors to be used as active neural sensors. In particular, the present invention relates to a device comprising an array of transducers or sensors as well as to a method for operating said interface devices.

Finally, the present invention also relates to the use of such interface devices in the in vitro or the in vivo fields.

### State of the art

There are two main methods for recording cell activity using large cellular (neural) interfaces comprising at least 128x128 sensors.

A first approach is based on a scanning method of the entire array of sensors.

One example of this method is disclosed in "Eversmann et al., 'A 128 x 128 CMOS Biosensor Array for Extracellular Recording of Neural Activity', IEEE Journal of Solid State Circuits, Vol.38, No.12, Dec. 2003." In this document, the authors describe a method and a device in which there are 128 rows and 128 columns. The entire array of sensors is individually scanned at a sufficient scanning frequency (2 kiloframes per second, (kfps)). The scanning is done with a row and column addressing technique. Each row has one amplifier leading to a total of 128 amplifiers. These are further multiplexed 8 to 1 and thus there are 16 outputs. This information is sent off the chip to a PC where 16 A/D converters are used and the information is processed.

The amount of information to be processed is quite large, 32 MS/s (MegaSamples/second). This raises important problems for post processing of the information. As nowadays arrays get larger the amount of data to be processed also increases. An array of 256x256 sensors would require 131 MS/s. Furthermore, the noise is also a concern as a recording amplifier has to be present in every sensor, the size of which has to be limited to keep the pitch of the sensor within a reasonable value; comparable to the size of a neuron (between about 20µm and about 100µm).

The second method is based on the assumption that not all sensors are in contact with a cell (neuron) and are therefore not of interest.

An example of this method is disclosed in "U.Frey et al., 'An 11k-electrode 126-channel high density microelectrode array to interact with electrogenic cells,' in ISSCC 2007, San Francisco, California, February 2007, pp. 158 - 159", wherein, the authors describe a very large array with 11,000 sensors having 126 channels that are permanently routed to sensors of interest. The amount of data is consequently reduced and noise performance is improved as the sensors only require a routing circuitry.

Because of the scarcity of neurons on the sensors, the system is capable of recording most of the cellular activity. Nevertheless, all of the neurons are not observable, and the device is always limited to the number of channels available for routing. In this case, only 1.14 % of the array is observable at any one time. Increasing the number of sensors will require increase in the number of channels. Assuming a linear increase, for a 256x256 array, 504 channels will be required for the same system monitoring capability.

Document US2004/0106101 discloses a system and a method for quality control of shipped neural cell culture on a micro electrode array. In such a system, the active channels are recorded before an event such as shipping and controlled after the event. The disclosed system is not suitable for real-time detection and routing of the active channels.

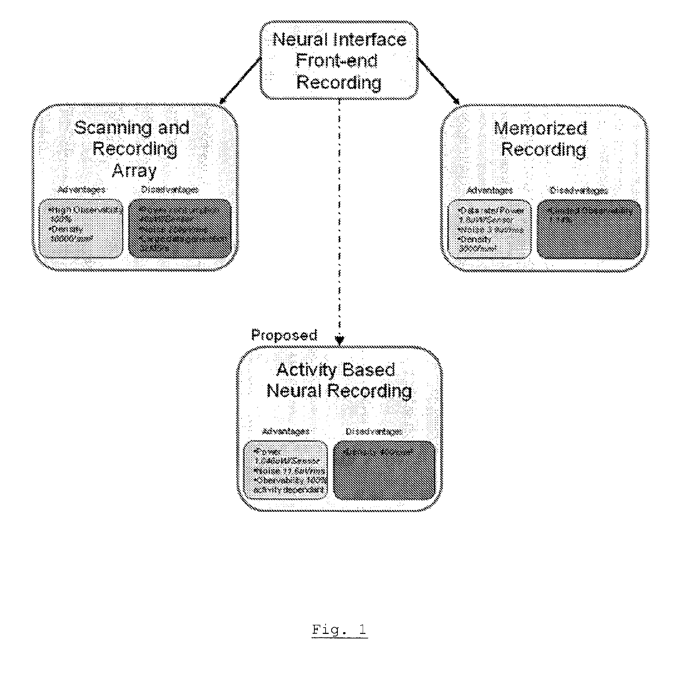

An overview of the methods used according to the state of the art is represented in Figure 1.

### Summary of the invention

The present invention relates to an active interface device for detection and recording of cell activity, in particular in neural systems, comprising:
- a transducer or sensor array comprising a plurality of transducers or sensors arranged to transform a cell activity into an electrical signal;
- at least one detection unit for detecting the electrical signal(s);
- at least one recording unit for recording the electrical signal(s), comprising a plurality of recording channels arranged for being routed to said transducers or sensors;
- at least one control unit;
wherein said control unit is arranged for addressing said transducers or sensors to the detection unit(s), for activating transducers or sensors, and for routing the recording channels to (said) activated transducers or sensors.

The present invention further relates to an active interface device for detection and recording of cell activity, in particular in neural systems, comprising:
- a transducer or sensor array comprising a plurality of transducers or sensors arranged to transform a cell activity into an electrical signal;
- at least one detection unit for detecting the electrical signal(s);
- at least one recording unit for recording the electrical signal(s), comprising a plurality of recording channels arranged for being routed to said transducers or sensors;
- at least one control unit;
   wherein said control unit is arranged for addressing said transducers or sensors to the detection unit(s), for activating transducers or sensors, and for deciding upon detection of cell activity by said activated transducers or sensors to route the recording channels to those activated transducers or sensors which have detected cell activity (and/or generated an electrical signal).

Said transducer or sensor can be considered as a pixel in a network (array).

Advantageously, the device, according to the present invention, comprises at least two amplifying stages (or steps) for amplifying said electrical signal(s) wherein the first stage is performed by first amplifying means being present in the detection unit, the second stage is performed by second amplifying means being present in the recording unit.

Advantageously still, the device according to the invention comprises at least two amplifying blocks or units for amplifying said electrical signal(s) wherein a first amplification is performed by first amplifying means being present in the detection unit, and a second amplification step is performed by second amplifying means being present in the recording unit.

Alternatively, the device according to the invention comprises at least one amplifying block or unit for amplifying said electrical signal(s) wherein amplification of the electrical signal(s) is performed by (first) amplifying means being present in the detection unit.

Preferably, the device, according to the present invention, has a ratio of the gain between the first amplifying means and the second amplifying means which is at least of 5 and preferably superior to 10.

Preferably, the first amplifying means are faster than the second amplifying means.

Preferably, the first amplifying means are generating more noise than the second amplifying means.

In particular, the first amplifying means is being defined by a high gain, for a noise being comprised between 20µVrms-100µVrms and a bandwidth between 1MHz-3MHz.

In particular, the second amplifying means is being defined by a low gain, for a noise being comprised between 3µVrms-15µVrms and a bandwidth between 5 KHz-10KHz.

Preferably, said detection unit comprises (first) amplifying means arranged for amplifying said electrical signal.

Preferably, said detection unit comprises at least one comparator arranged to compare said amplified electrical signal with a predetermined threshold signal. Preferably, cell activity is detected if the amplified electrical signal is above the predetermined threshold signal.

Preferably, each transducer or sensor comprises at least one buffer.

Preferably, said detection unit comprises at least one sample and hold capacitor.

Preferably, said recording unit comprises (second) amplifying means.

Preferably, said (first and/or second) amplifying means are arranged to comprise at least one amplification stage.

Preferably, said at least one amplification stage comprises at least one low noise amplifier.

Preferably, the device according to the present invention further comprises at least one stimulation unit arranged for sending electrical stimulation signals to at least one transducer of the transducer or sensor array.

Preferably, the device according to the present invention further comprises a post-processing unit.

Preferably, said control unit comprises an on-chip controller.

Preferably, said control unit comprises a Field-Programmable Gate Array.

Preferably, the transducer or sensor array is divided into transducer or sensor sub arrays.

Preferably, each transducer or sensor subarray comprises at least one detector unit.

Preferably, each transducer or sensor subarray comprises at least one recording unit.

Preferably, each transducer or sensor subarray comprises at least one control unit.

Preferably, each transducer or sensor subarray comprises at least one stimulation unit.

Preferably, each transducer or sensor subarray comprises at least one post-processing unit.

The present invention also relates to a method for recording cell activity with a transducer or sensor array comprising a plurality of transducers or sensors, wherein, for each transducer or sensor, the following steps are performed:
a) addressing a transducer of the transducer or sensor array or addressing a transducer of a predetermined subset of transducers or sensors of the transducer or sensor array;
b) activating said transducer or sensor;
c) receiving from said activated transducer or sensor any electrical signal (possibly resulting from cell activity);
d) amplifying said electrical signal;
e) comparing said electrical signal to a predetermined threshold signal;
f) determining by using the comparison result of step e) that said electrical signal corresponds to a cell activity;
g) for any electrical signal corresponding to a cell activity routing a recording channel to said activated transducer or sensor (which has detected cell activity and/or generated an electrical signal);
h) recording said electrical signal from said activated transducer or sensor.

Preferably, in the method according to the present invention, the amplifying step d) comprises at least two amplifying stages (or steps) performed with a first amplifying stage (or means) having a high gain, in the detection stage defined here above as steps a) to f), followed by a second amplifying stage at a low gain performed during the recording stage defined here above as steps g) to h).

Alternatively, in the method according to the present invention, the amplifying step d) comprises at least one amplifying step (or stage) performed with first amplifying means, preferably having a high gain, in the detection stage defined here above as steps a) to f). Optionally, and depending on the outcome of determination step f), the amplifying step (or stage) performed with first amplifying means, may or may not be followed a by a second amplifying step, preferably having a low gain, performed during the recording stage defined here above as steps g) to h).

The present invention further relates to a method for recording cell activity with a transducer or sensor array comprising a plurality of transducers or sensors, wherein, for each transducer or sensor, the following steps are performed:
a) addressing a transducer of the transducer or sensor array or addressing a transducer of a predetermined subset of transducers or sensors of the transducer or sensor array;
b) activating said transducer or sensor;
c) receiving from said activated transducer or sensor any electrical signal (possibly resulting from cell activity);
d) amplifying said electrical signal, preferably with first amplifying means;
e) comparing said electrical signal to a predetermined threshold signal;
f) determining by using the comparison result of step e) that said electrical signal corresponds to a cell activity;
g) for any electrical signal corresponding to a cell activity routing a recording channel to said activated transducer or sensor which has detected cell activity (and/or generated an electrical signal);
h) amplifying said routed electrical signal, preferably with second amplifying means; and
i) recording said amplified electrical signal from said activated transducer or sensor.

Preferably, in the method according to the present invention, the ratio of the gain between the first amplifying stage and the second amplifying stage is at least of 5 and preferably superior to 10.

Preferably, the sequence of steps a) to h) for the next or subsequent transducer or sensor starts between steps c) and d).

Preferably, the step a) to h) are repeated for the next or subsequent transducer or sensor.

Preferably, the method according to the present invention further comprises - after step h) - the step of sending sequentially the recorded electrical signals (or recorded cell activity) to a post-processing unit.

Preferably, the step of comparing is performed by a detection unit.

Preferably, the step of determining - if the acquired electrical signal corresponds to cell activity - is performed by a control unit.

Preferably, the step of routing is performed by the control unit.

Preferably, the step of recording is performed by a recording unit.

### Brief description of the drawings

Figure 1 is representing a comparison of the state of the art with the new proposed interface device for neural front-end recording system.

Figure 2 is representing a block diagram of the several components of an interface device according to the present invention.

Figure 3 is representing a specific embodiment of such an interface device as generally described in Figure 2 wherein the array of transducer/sensor is in the form of M x N sub arrays.

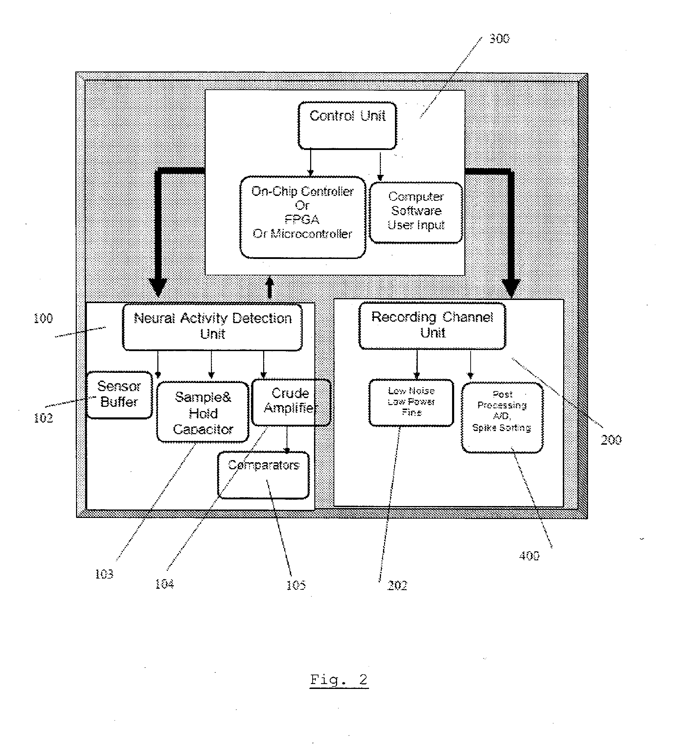

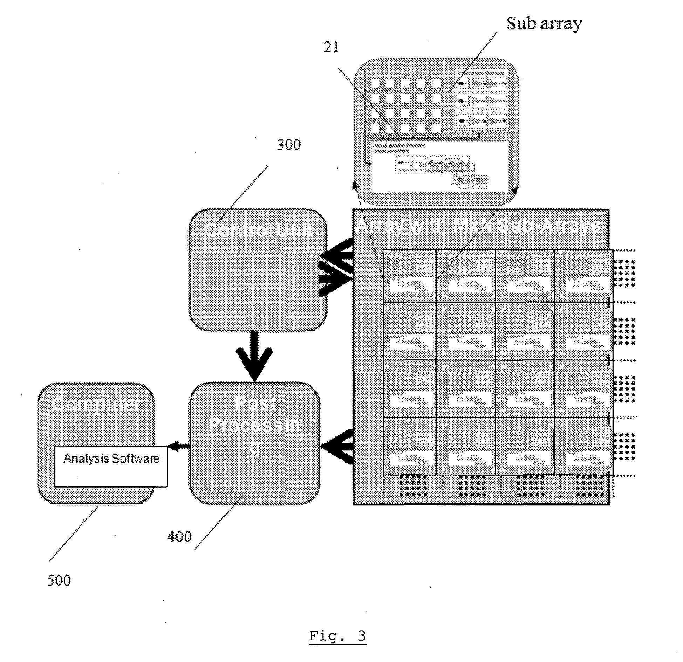

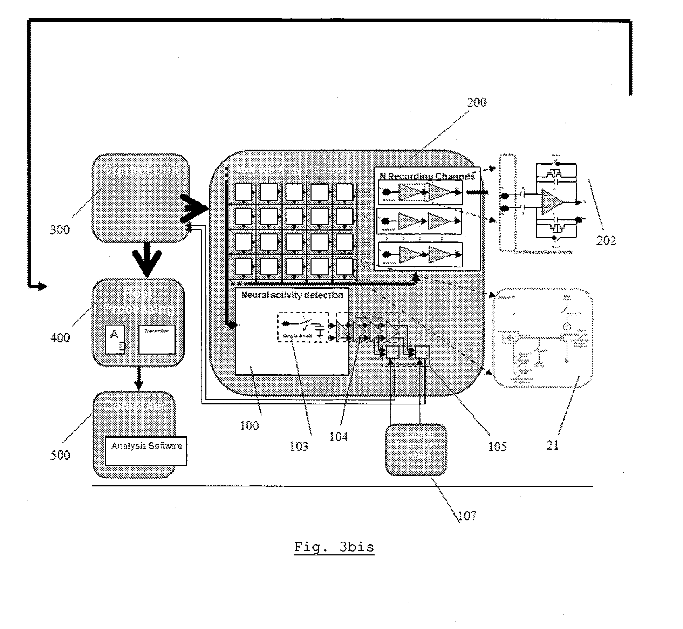

Figure 3bis is representing a embodiment of the components of the transducer/sensor array and corresponding to the schema block of Figure 2.

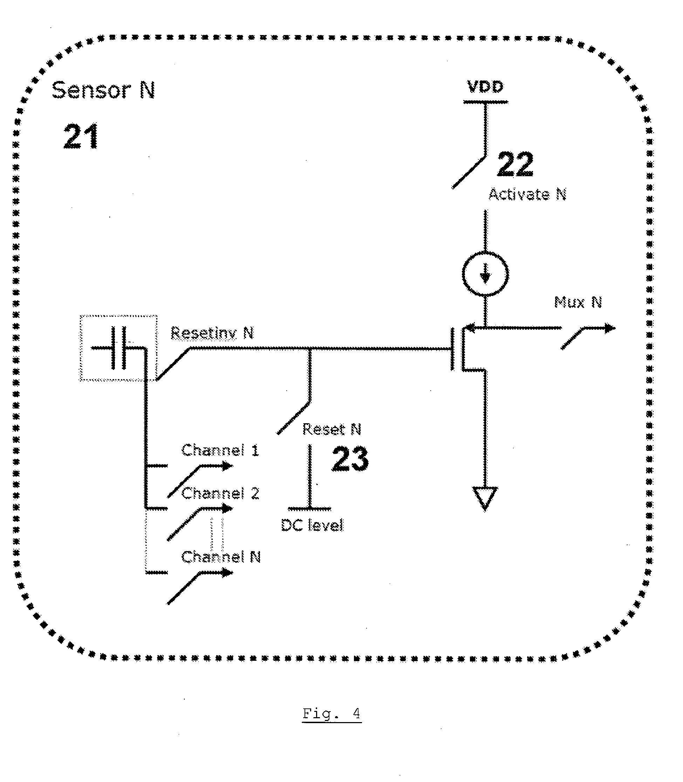

Figure 4 is representing an example of a transducer/sensor.

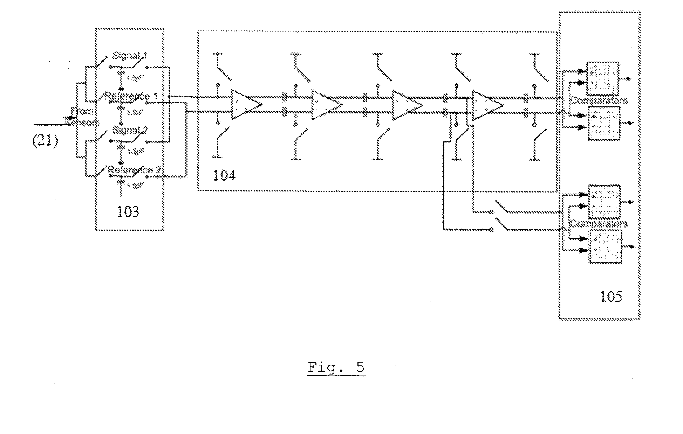

Figure 5 is representing an example of a detection unit used in the present invention.

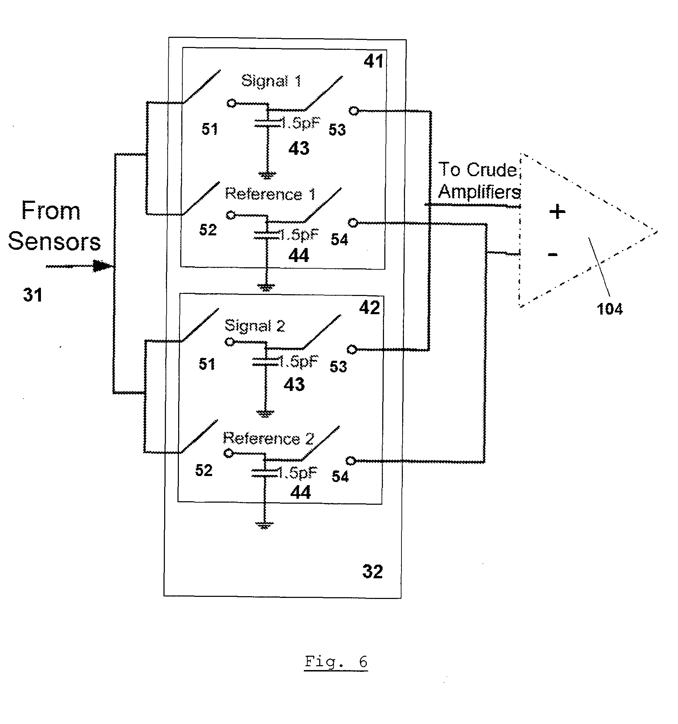

Figure 6 is representing an example of sample and hold capacitors of the detection unit.

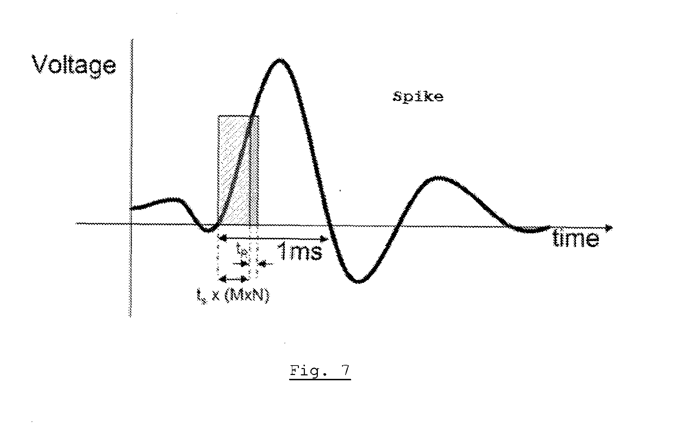

Figure 7 is representing an example of the scanning pulse of a cell activity (neural spike) which is a representation of the voltage versus line.

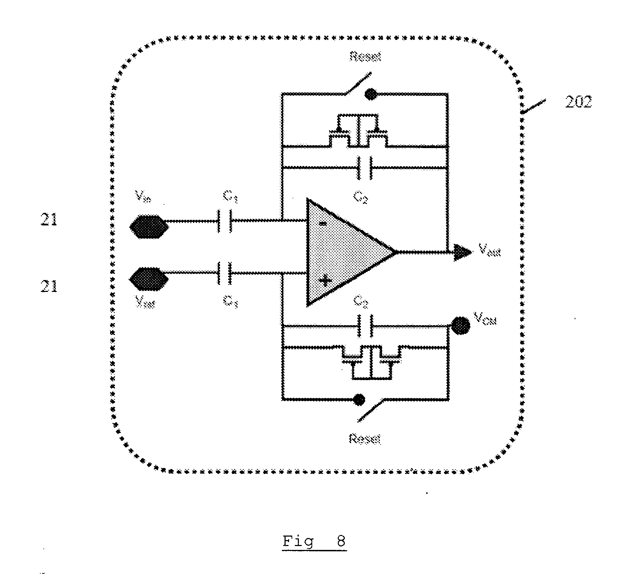

Figure 8 is representing an example of a low noise and low consumption fine amplifier.

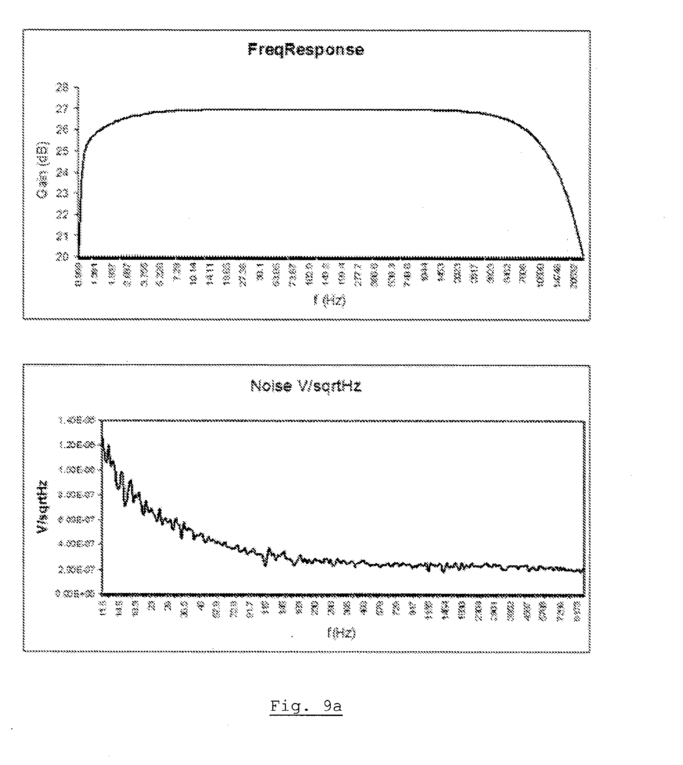

Figure 9a is representing an example of frequency response and noise measurements of a fine amplifier comprised in the recording unit according to the present invention.

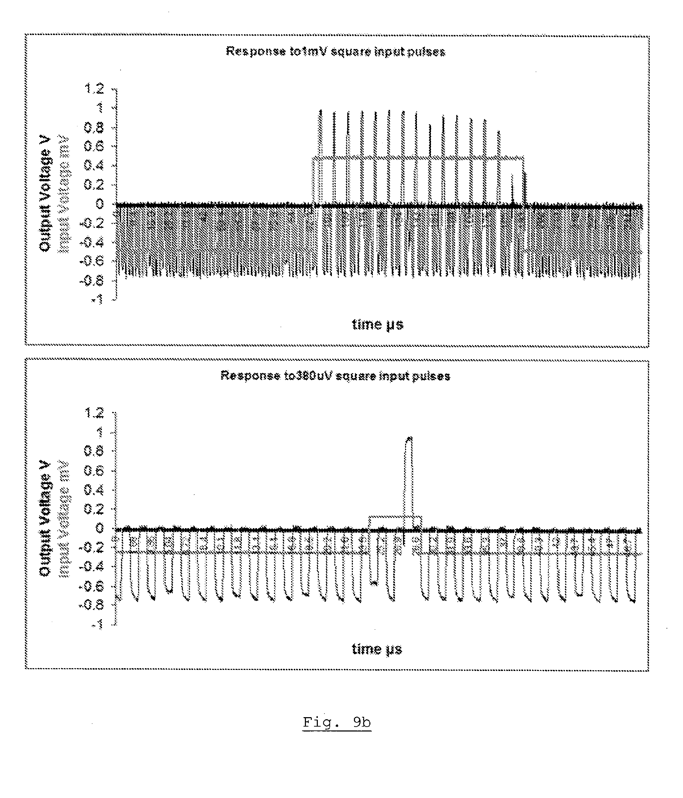

Figure 9b is representing an example of the output from a crude amplifier comprised in the detection unit according to the present invention.

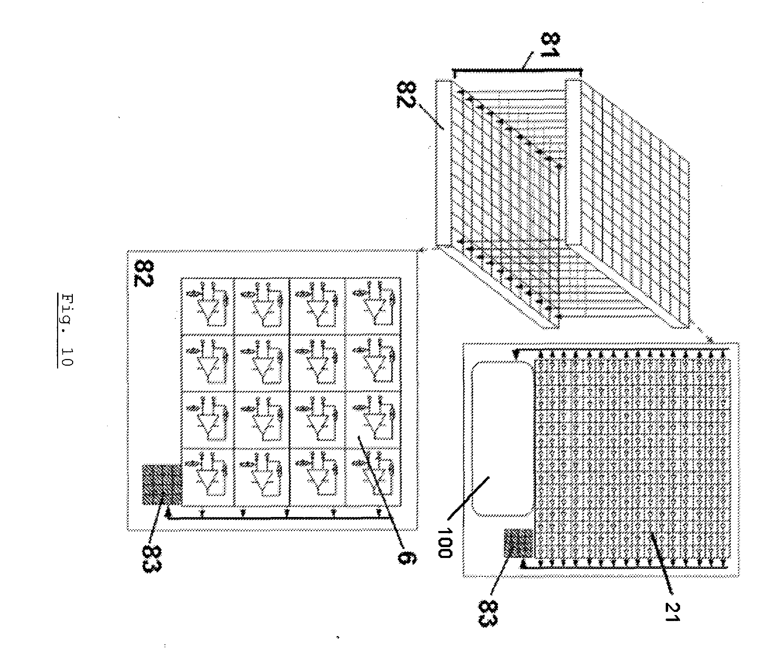

Figure 10 is representing an example using 3D integration technology accordingly to the present invention.

### General description of the invention

According to a first aspect, the present invention is related to a sensor array having a plurality of sensors arranged for stimulating neurons and recording neural activity. The network comprises a first amplifying block or unit and a second amplifying block or unit and (with) a digital control block (unit). The first amplifying block is arranged for detecting neural activity. The control block (unit) is arranged for addressing the sensors, for routing the channels to these sensors and for activating the second amplifying block. The second amplifying block is arranged for recording the neural activity.

An intelligent real-time routing system is provided that allocates available resources or channels based on neural activity. The network of the present invention can comprise a sensor array which is scanned with a first amplifying means (also called block) or crude circuitry which is optimized for area (surface) and very high gain and high speed for the detection of neural activity.

Once activity at a sensor or pixel or a set of sensors or pixels is detected, an available channel within the array is routed by the control block (unit) to the corresponding sensor pixel for recording the signal.

The control block (unit) can designate a set of sensors as one block in case a neuron is present on more than one sensors or its signal is being received at neighboring sensors.

Recording can be performed via one of the free recording channels within the array by the second amplifying block or (also called) the fine amplifier.

The recording channel can comprise of fine amplifiers divided into stages which are present within the array.

Further, the recording channel can comprise of any post processing like A/D converters or "spike" filtering which is preferably present outside the array.

The fine amplifiers are preferably optimized for low power, low noise and area.

The recording from the recording channel can tell if the detection of neural activity (spike) by the neural activity detection system is true or false. The probability of a false detection and hence a wrong routing of a channel depends on the amplitude of the signal versus the noise in the neural activity detection unit.

The recording channels are accordingly resources to record neural activity. These resources are preferably allocated based on need in real time. By intelligently using the limited channels, activity can be recorded from the entire sensor array, because there is no need to permanently route any channel to a given sensor. At the same time, the generation of large amounts of data and the power consumption can be minimized because the recording channels are not used needlessly all the time when there is no activity.

Preferably, the sensor network is an active, front-end interface device arranged for scanning and recording a transducer and/or sensor array and arranged for sending the recorded data (being the useful data) to a post-processing block. Recording of data is only performed when neuron activity is detected. Accordingly, only a limited amount of data needs to be forwarded to a post-processing block.

In an preferred embodiment, the sensor network is arranged in a 3D integrated system. A plurality of sensor networks can be implemented on several top dies. The bottom die has a number of recording channels. The number of channels is preferably based on the available area in the bottom die. The sensors on the top die can be routed to any of the recording channels on the bottom die through the 3D vias. This way, given that the area is fixed, a larger number of channels are available for each layer or sub-array than would be if only a single die would be used.

These same channels can also be used for stimulation, if a stimulation circuitry is placed on the bottom die. Both dies can be made in separate technologies to optimize the readout and reduce costs.

The present invention will be described with respect to particular embodiments and with reference to specific drawings but the invention is not limited thereto but only by the claims.

The drawings described are only schematic and are non-limiting.

In the drawings, the size of some of the elements may be exaggerated and not drawn on scale for illustrative purposes. The dimensions and the relative dimensions do not necessarily correspond to actual reductions to practice of the invention.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the invention described herein can operate in other orientations than described or illustrated herein.

The term "comprising", used in the claims, should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting of only components A and B. It means that with respect to the present invention, the only relevant components of the device are A and B.

An interface device according to the present invention is displayed as a schema block in Figure 2.

Figure 3 and 3bis are representing an implementation of a specific embodiment for M x N sub array of transducers with neural activity detection for N recording channels.

Figures 4, 5 and 6 are representing in details particular embodiments of constituting elements of the devices as represented in Figures 2, 3 and 3bis. In particular, Figure 4 is displaying an example of a transducer or sensor used for neural detection.

The device according to the present invention, in particular as described in Figures 2, 3 and 3bis, comprises a transducer array comprising a plurality of transducers, at least one detection unit (100) arranged for detecting cell activity, at least one recording unit (200) arranged for recording cell activity and comprising a plurality of recording channels, and at least one control unit (300) for controlling the detection unit(s), the recording channel(s) and the recording unit(s).

In the present invention, the term 'transducer' refers to any device arranged for transforming cell activity into an electrical signal.

In the present invention, the term 'cell activity' refers to any signal produced by the cell, irrespective of the nature of the signal. The signal can be a mechanical stress, a release of ions, a release of neurotransmitters, an electrical signal, etc.

Preferably, the detection unit (100) comprises amplifying means (104) arranged for amplifying electrical signals resulting from cell activity.

Accordingly to a preferred embodiment as represented in Figure 2 and in particular in Figure 5, the detection unit (100) comprises at least the following elements sample and hold capacitor(s) (103), crude amplifier(s) (104) and comparator(s) (105).

The threshold voltage defines at which input voltage, the comparator will give the logical value 1. If the threshold voltage is set below the noise at the output of the "crude" amplifier means, the comparator will be triggered with this noise. So, the threshold voltage is at a level above this noise at the output of the "crude" amplifier means. Usually noise is defined as input referred, meaning that the noise would be at the input of the "crude" amplifier means rather than the output. But to make it more simple lets just say that the noise is present at the output of the "crude" amplifier means.

Accordingly, said detection unit (100) comprises a first amplifying stage (or step) (104) also called hereunder "crude" amplifying stage (or step).

Preferably, the recording channel unit (200) is comprising a second amplifying stage (or step) (202) also called hereunder "fine" amplifying stage (or step).

The detection unit (100) is arranged for comparing the electrical signal coming from a transducer (21) to a reference signal - called reference threshold signal (107)- and is further arranged to send the comparison result to the control unit (300).

An example of transducer or sensor (21) as displayed at Figure 4.

For example, the transducers are sensors (21) suitable for electrophysiological measurements such as planar electrodes on an insulating glass substrate.

For example, the sensors can be electrochemical sensors based on enzymatic catalysis of a reaction that produces or consumes electrons, namely redox enzymes.

Other examples of suitable sensors are CMOS based sensors.

Another example of sensors are piezoelectric sensors.

Preferably, each transducer of the transducer or sensor (21) array comprises at least one buffer (represented as 102 in Figure 2).

In the present invention, the term 'buffer' can refer to a device that provides impedance transformation from one circuit to another.

Preferably, said buffer(s) is (are) implemented as gated source followers.

Preferably, each transducer or sensor (21) is activated sequentially by the control unit (300).

Preferably, the transducer or sensor (21) can be either in an active state or in an inactive state.

The term 'active state' refers to a state wherein the transducer or sensor (21) is able to transform cell activity into an electrical signal.

The term 'inactive state' refers to a state wherein the transducer or sensor (21) is not able to transform cell activity into an electrical signal.

The state of the transducer or sensor is controlled by the control unit (300).

The transient activation of a transducer or sensor reduces power consumption and avoid heating the cells which are on top of the transducer or sensor.

The transducer or sensor (21) as displayed at Figure 4 can be addressed using a set of addressing (activation) switches (22).

The transducer or sensor (21) as displayed at Figure 4 can be activated by turning on the switch (22). The switch (22) has two states: ON and OFF.

Preferably, said electrical signal is amplified (in 104) prior to the comparison (in 105) with the reference threshold signal through e.g. an external threshold setting (107) as represented in Figure 3bis.

The result of said comparison is sent to one of the at least one control unit (300).

The detection threshold (107) for the comparators can be set externally by setting the reference voltage of the comparator to an predetermined level above the noise.

For example, if the noise is given by σ, then an acceptable interval of input noise can be 20 µVrms < σ < 100 µVrms for a possible reference voltage of 1σ∼5σ (input noise multiplied by the gain of the crude amplifier).

If this noise is σ. Then the acceptable interval of threshold is 1σ to 5σ, meaning 1 times σ or 5 times σ.

If the input referred noise is 100µrms then output referred noise σ can be calculated as 100µVrms.G where G is the Gain of the "crude" amplifier means.

The intervals for the input referred noise are comprised between 20µVrms to 100µVrms for a "spike" (cell activity) between 100µV to 5mV. Typically, as represented in Figure 7, a spike has a duration of 1ms.

Typically, the noise for "crude" amplifier means is comprised between 20µVrms-100µVrms while the noise for the "fine" amplifier means is comprised between 3µVrms-15µVrms.

Preferably, for a bandwidth comprised between 1MHz-3MHz for the "crude" amplifier means and between 5-10KHz for the "fine" amplifier means.

The power of the "crude" amplifier means is comprised between 100µV and 200µV while the power for the "fine" amplifier means is comprised between 5µV and 15µV.

The "crude" amplifier means (104) with conjunction with the comparator (105) gives a signal if there is cell activity (presence of a spike as in Figure 7). Sometimes the comparator (105) will be triggered by noise in the "crude" amplifier means (104) itself. The "crude" amplifier means (104) can be also non-linear

The "fine" amplifier means is connected when there is cell activity. It is the noise, and the output of the "fine" amplifier means which will decide if the activity detected with the help of the "crude" amplifier means (104), the comparators (105) and the buffers (102) in the sensors, is an actual "spike" or is due only to detected noise.

An example of "fine" amplifier means is given in Figure 8.

Preferably, the detection unit (100) comprises (sensors) buffers (102) for driving the sample and hold capacitors (103) as represented in Figure 5.

Preferably, the detection unit (100) comprises sample and hold capacitor(s) (103) for the implementation of Correlated Double Sampling (CDS) for removing the offset in the buffers of the transducers and to remove 1/f noise in these buffers.

Preferably, said buffers (102) are arranged for driving the sample and hold capacitors (103).

Preferably, said buffers (102) are implemented as source followers.

An example of sample and hold capacitors (103) is shown in Figure 6.

In this example, a detection unit (100) comprises two pairs of sample and hold capacitors (41,42) comprising upstream switches (51,52) and downstream switches (53,54). These switches are controlled by the control unit (300).

Each pair of sample and hold capacitors has a signal capacitor (43) and a reset capacitor (44) which are arranged to remove the offset in the source followers.

As shown in Figure 6, the signal capacitor (43) samples and holds the signal plus the offset present in the source follower while the reference capacitor (44) holds just the offset by resetting the source follower by activating the reset switch (23) as shown in Figure 4. This helps to remove the offset.

The presence of two pairs of sample and hold capacitors (41, 42) reduces power consumption. Indeed, one pair of capacitors samples the signal from the currently addressed (*tᵢ₊₁*) transducer or sensor while the other pair holds the value of the previously addressed transducer (*tᵢ*) or sensor for the "crude" amplifier(s)(104).

Preferably, the "crude" amplifier means (104) are designed for high bandwidth, high gain and area and compromised on noise and gain error since the primary function of these is not to record the activity but just to detect it.

Preferably, the amplifiers (104) are designed for low area (surface) such that the average pitch of the transducers or sensors (21), including the distance between the "crude" amplifier means (104) and the recording channels (200), does not exceed 50µm.

The detection of an electrical signal resulting from cell activity requires very high gain to overcome the mismatch in the comparators.

In the present invention, the highest gain needed refers to the ratio between the value of the estimated mismatch in the comparator and the value of the smallest signal that has to be detected.

The offset in the amplifiers can be removed by output offset cancellation.

Alternatively, the offset can be removed by input offset cancellation.

The control unit (300) is arranged for addressing a transducer or a sensor (21) of the transducer array (M x N) to a detection unit (100).

The control unit (300) is arranged to activate a transducer or a sensor (21) subsequently to its addressing.

The control unit (300) can activate sequentially each transducer or a sensor (21) of the transducer array.

The control unit (300) can activate sequentially each transducer or a sensor (21) belonging to a predetermined subset of transducers.

In the present invention, the term 'next' or 'subsequent' preferably refers to the a temporal sequence.

Preferably, the control unit (300) is arranged to determine, based on the result of the comparison made by the detection unit, if a transducer or a sensor (21) has received from a transducer or a sensor (21) an electrical signal resulting from cell activity.

The control unit (300) is further arranged for routing (connecting) a recording channel to a transducer or sensor for which a cell activity has been detected accordingly to said comparison.

Preferably, the control unit (300) is preferably further arranged for addressing the transducers or sensors, for activating the transducers or sensors (21) and for routing the recording channels (200).

The recorded signal of this recording channel will confirm if the signal detection by the detection unit (100) was caused by cell activity or by the noise in the detection unit (100).

The externally set reference (107) of the comparator (105) in the detection unit (100) will control how much the detection unit (100) is sensitive to noise in the detection unit (100).

Preferably, the control unit (300) comprises an on chip controller, a FPGA or any other microcontroller known to the person skilled in the art.

The probability of a false detection and therefore of wrong channel's routing depends on the ratio between the amplitude of the signal and the mean amplitude of the noise of the detection unit (100).

Preferably, the recording channel unit (200) comprises at least one recording channel (and preferably N channels).

Preferably, each recording channel unit (200) is arranged for recording the signal of one sensor at any given time.

Preferably, the recording channel unit (200) further comprises amplifying means (202) so called "fine amplifying" means.

Preferably, each recording channel of the recording channel unit (200) comprises at least one stage of amplification.

Preferably, each stage comprising at least one low noise and low power consumption fine amplifier.

Preferably, the amplifying means (104) of the detection unit (100) are faster than the amplifying means (203) of recording channel unit (200).

Preferably, the amplifying means (104) of the detection unit (100) generates more noise than the amplifying means (202) of the recording channel unit (200).

Preferably, the term 'low noise fine amplifier' refers to an amplifier having a noise level between 2 µVᵣₘₛ and 15 µVᵣₘₛ.

Preferably, the term 'low power consumption fine amplifier' refers to an amplifier consuming between 3 µW and 35 µW.

The amplifier comprises two inputs. The first input (Vᵢₙ) is connected to the transducer or the sensor (21) from which an electrical signal is to be recorded, whereas the second input (V_{ref}) is connected to a transducer or a sensor (21) defined as reference transducer or sensor (21). This allows differential recording and reduces the effect of external noise.

Examples of frequency response of the fine amplifier as well as example of noise measurements are shown in Figure 9a.

Examples of measurements of the detection unit are shown in Figure 9b.

Figure 9b shows the output of the "crude" amplifier means after a square wave is given as an input to one of the transducer or the sensor (21) through a capacitively coupled input. The scanning is being done between four transducers or sensors (21).

Preferably, the transducer array or sensor (21) array comprises at least one stimulation unit.

Preferably the stimulation can be done on each transducer or sensor (21) individually.

Preferably, each stimulation unit uses the recording channels for sending stimulation signals to the transducers or sensors (21).

In the present invention, the term 'stimulation signal' refers to any electrical signal generated by the stimulation unit leading to the generation of cell activity by a cell or a group of cells.

Preferably, the device according to the present invention also comprises a post-processing unit (400).

The post-processing unit (400) is arranged for processing the recorded data.

For example, the recorded data is converted into a digital signal by an Analog-to-Digital converter before being transmitted to an external device (500) e.g. computer for analysing the recorded information.

Another aspect of the present invention relates to a method for detecting and recording cell activity by means of a transducer array or sensor (21) array comprising a plurality of transducers or sensors.

The present invention also relates to a method that routes recording channel(s) to transducer(s) or sensor(s) (21) for which a cell activity has been detected.

The recording channels are resources at the disposal of the transducer or sensor (21) array to record cell activity. These resources are allocated based on real-time needs. By intelligently using the limited number channels, activity can be recorded from the entire transducer or sensor (21) array as there is no need to permanently route any channel to a given sensor. Moreover, there is no need to generate large amounts of data and power consumption is saved, as there is no use of the recording channels needlessly all the time when there is no activity.

Preferably, the control unit is arranged for generating control signals for controlling the detection and the recording channels units (100 and 200).

Whereby the control signals for controlling the detection unit (100) comprise signals for turning on and addressing the sensor buffer (102) and resetting the sensor buffer. The control signals further comprise signals for controlling the connection of the currently addressed sensor buffer (102) to the sample and hold capacitor(s) (103) and the connection of the sample and hold capacitor(s) to the "crude" amplifier(s) (104) as well as the timing of these connections.

The control signals further comprise signals for controlling the removal of offset of the "crude" amplifier means (104).

Preferably, the control signals are arranged as clocks for the comparator (105) present after the "crude" amplifier means (104).

The control signals for controlling the recording unit comprise activation signals for turning on the recording channel (200) and resetting the amplification stages (104) and for connecting the recording unit (200) to the necessary sensors.

Preferably, the step of generating control signals for controlling the detection further comprises sending signals for turning on and addressing the sensor buffer (102). The step further comprises connecting the addressed sensor buffer (102) to the sample and hold capacitors (103) and connecting the sample and hold capacitors (103) to the amplifying means (104).

Preferably, the step of generating control signals for controlling the recording further comprises sending signals for turning on the recording channel (200) and resetting the amplification stages (202) and for connecting the recording unit to the necessary sensors.

Preferably, the step of activating each transducer or sensor (21) of a plurality (array) comprises activating each transducer or sensor (21) sequentially.

Preferably, the step of activating transducer or each sensor (21) of the plurality comprises activating each transducer or sensor (21) of a predefined subset of transducers or sensors (21). Said subset can be programmed in advance into the control unit (300). Said subset can also be defined in real-time meaning that the control unit (300) is learning and deciding itself how the patterns are changing or should be changed.

After performing the comparison, the comparison result is sent to the control unit (300).

This process is repeated for each transducer or sensor (21) of the plurality (array).

The cell activity or "spike" is illustrated in Figure 7, where the voltage versus the time required to scan is shown (scanning performed by detection unit). In an example such a "spike has a typical duration of 1 ms. This time is given by *tₛx(MxN)* being the time spent on one transducer or sensor (21), whereby tₛ is the scanning time and *(MxN)* represents the number of sensors to be scanned. This number can be the total number of sensors of an array or can represent the number of sensors to be scanned according to a predefined subset. Such pattern can be defined and memorized in the control unit before the detection and recording process or can be defined in real-time during the scanning process and adapted according to the detection results.

The scanning of the transducer or sensor array(21) or of a subset of transducer or sensor (21) of the array lasts for a period inferior to a half of the time of a "spike", to ensure that no activity is missed and that the peak of the "spike" can be recorded.

According to another embodiment, the transducer or sensor array (21 is arranged in a 3D integrated system as illustrated in Figure 10.

A plurality of transducer or sensors arrays (21) can be implemented on several top dies (81).

The bottom die (82) has a number of recording channels. The number of recording channels is preferably based on the available area in the bottom die.

The transducers or sensors (21) on the top die can be routed to any of the recording channels on the bottom die through the 3D vias (83).

This way, given that the area is fixed, a larger number of recording channels are available for each layer or sub-array than would be if only a single die would be used.

In a preferred embodiment, two dies are connected to each other using 3D through via Arrays. The top die has an *XxY* array comprised of *MxN* subarrays of transducers. The bottom die has *N* recording channels. The number of channels is based on the available area in the bottom die. The transducers or sensors (21) on the top die can be routed to any of the *N* channels on the bottom die through the 3D vias. This way, given that the area is fixed, a larger number of channels are available for each subarray than would be if only a single die would be used. The recording channels can also be used for stimulation, by placing stimulation circuitry (stimulation unit) on the bottom die. Both dies can be made in separate technologies to optimize the readout and reduce costs.

According to a preferred embodiment of the present invention, the transducer or sensor (21) array has been implemented in the form of a 16x16 array in AMIS 0.35µm technology. The array has a detection unit comprising buffers inside the transducer or sensor (21), sample and hold capacitors, crude amplification system and comparators as mentioned above. If a cell activity occurs crude amplifiers in conjunction with the buffers and the sample and hold will amplify this signal and the comparators will generate a logical signal. This will be detected by the control unit (300) which will route an available recording channel to the respective transducer or sensor (21). The recording channel is a first stage low noise and low power, fine amplifier. The control unit(300) is represented by an external FPGA which sends control signals to the chip. A neural spike lasts for about 1ms. The detection unit scans through the array with in 410ps. The recording channels are reset with in 40µs. Therefore the in the worst case scenario, 450µs out of 1ms of the spike will be missed. This is within the target of less than half in order to be able to record the peak.

In one embodiment of the present invention, the transducer or sensor (21) array are arranged to form a matrix comprising *X* columns and *Y* rows.

Preferably, the transducer or sensor (21) array is divided in *S* transducer or sensors (21) subarrays.

Preferably, each subarray *i* (*i*=*1,...S*) comprises M*ᵢ* columns and *Nᵢ* rows.

More preferably, the sum of M*ᵢ* (*i=1,...S*) is equal to *X.*

More preferably, the sum of *Nᵢ* (*i=1,...S*) is equal to *Y.*

Preferably, each subarray *i* comprises a detection unit.

Preferably, each subarray *i* comprises a recording unit.

Preferably, the transducer or sensor (21) array comprises at least one control unit.

Preferably, each subarray *i* comprises a control unit.

Preferably, the transducer or sensor (21) array further comprises post-processing means.

Preferably, the transducer or sensor (21) array further comprises at least one stimulation unit for sending stimulation signals to said transducer or sensor.

### Detailed description of preferred embodiments

According to preferred embodiments, the device of the present invention comprises a sensor array having a plurality of sensors arranged for stimulating neurons and recording neural activity.

The array comprises a first and a second amplifying block and a digital control block as presented in Figure 2. The first amplifying block is arranged for detecting neural activity in the form of a spike. The control unit is responsible for the addressing of the sensors for the neural activity detection system and to generate the necessary control signals as well as the routing information for the recording channels. The second amplifying block is arranged for recording the neural activity. Recording channels are designed for low noise recording of this neural activity. The basic concept is to allocate recording channels based on neural activity rather than permanently routing them or multiplexing and recording all the sensors and generating unnecessary data.

The first amplifying block or Neural Activity Detection system comprises of buffers present in the sensors, sample and hold capacitors, crude amplifiers and comparators as shown in Figure 2. The neural Activity Detection system quickly scans through the subarray to detect any spike occurring at any of the sensors in the array. The scanning is done in less than half the time of a spike, to ensure that no activity is missed and that the peak of the spike can be recorded. Figure 7 shows a spike, with the time required to scan shown. This time is given by tₛx(MxN) ((MxN- size of array), with tₛ being the time spent on one.

The second amplifying block or Neural Recording System comprises of low noise, low power fine amplifiers. This system may also comprise of a post-processing system. The control unit may comprise an on chip controller or an FPGA or any other microcontroller known to the person skilled in the art.

An example system has been implemented in the form of a 16x16 array in AMIS 0.35µm technology. The array has a neural activity system comprising buffers inside the sensors, sample and hold capacitors, crude amplification system and the comparators as mentioned above. If a neural activity occurs crude amplifiers in conjunction with the buffers and the sample and hold will amplify this signal and the comparators will generate a logical one. This will be detected by the control unit which route an available recording channel to the respective sensor. The recording channel is a first stage low noise and low power, fine amplifier. Control unit is represented by an external FPGA which sends control signals to the chip. A neural activity detection system scans through the array with in 410µs. The recording channels are reset with in 40µs; tₛ and tᵣ respectively in Figure 7. Therefore the in the worst case scenario, 450µs out of 1ms of the spike will be missed. This is within the target of less than half in order to be able to record the peak.

The buffers present in each sensor are implemented as gated source followers. The schematic of a sensor is shown in Figure 4. A sensor is only activated, by the Activate N switch as shown in Figure 9, when it is addressed. This helps to conserve power. The sample and hold capacitors are shown in Figure 6. There are two pairs of sample and hold capacitors. Each pair has a signal capacitor and a reset capacitor which is needed to remove the offset in the source followers. The signal capacitors samples and holds the signal plus the offset present in the source follower while the reference capacitor holds just the offset by resetting the source follower by activating the reset switch as shown in Figure 4. This helps to remove the offset. Having two pair of sample and hold capacitors helps to reduce power. One pair of capacitors samples the signal from the currently addressed sensor while the other pair holds the value of the previously addressed sensor for the crude amplifiers. The same complete time (1.6µs) is used to address a sensor, to sample the signal and the same complete time (1.6µs) is used to amplify the signal by the crude amplifiers. This reduces the bandwidth requirement to the source follower as well as the crude amplifiers as well as the comparators. This in turn helps to conserve power at the cost of some area from the two extra capacitors.

The crude amplifiers are designed for high bandwidth, high gain and area and compromised on noise and gain error since the primary function of these is not to record the activity but just to detect it. High gain is required to overcome the mismatch in the comparators. The highest gain needed id defined by the estimated mismatch in the comparator divided by the smallest signal that has to be detected. The detection threshold for the comparators can be set externally, to an acceptable level above the noise. The offset in the amplifiers is removed by output offset cancellation. The crude amplifiers connected to the sample and hold capacitors are shown in Figure 5. The amplification can be selected as 57.8dB or 77.1dB by setting a switch.

The recording channel is represented by a first stage low noise, low power fine amplifier as shown in Figure 8. The gain of this amplifier is 24dB.

The power consumption is 9µW. Integrated noise between 100Hz-5kHz is 11.628µVrms. The frequency response of the fine amplifier as well as noise measurements are shown in Figure 9a. Measurements of the Neural Activity Detection system are shown in Figure 9b. It shows the output of the crude amplifiers after a square wave is given as an input to one of the sensors through a capacitively coupled input. The scanning is being done between four sensors.

The control unit is implemented in the form of a programmed FPGA.

Using said 3D integration capabilities the concept can be further expanded as shown in Figure 10. Two dies are connected to each other using 3D through via Arrays. The top die has a XxY Array comprised of MxN sub-arrays of sensors as mentioned above. The bottom die has N recording channels. The number of channels would be is based on the available area in the bottom die. The sensors on the top die can be routed to any of the N channels on the bottom die through the 3D vias. This way, given that the area is fixed, a. A larger number of channels are available for each sub-array than would be if only a single die would be used.

These same channels can also be used for stimulation, if a stimulation circuitry is placed on the bottom die. Both dies can be made in separate technologies to optimize the readout and reduce costs.

## Claims

1. An active interface device comprising:
- a transducer or sensor (21) array comprising a plurality of transducers or sensors (21) arranged to transform a cell activity into an electrical signal;
- at least one detection unit (100) for detecting the electrical signal(s);
- at least one recording unit (200) for recording the electrical signal(s), comprising a plurality of recording channels arranged for being routed to said transducers or sensors (21);
- at least one control unit (300);
wherein said control unit (300) is arranged for addressing said transducers or sensors (21) to the detection unit(s) (100), for activating transducers or sensors (21), and for deciding upon real-time detection of cell activity by said activated transducers or sensors to real-time route the recording channels to those activated transducers or sensors which have detected cell activity.

2. The device according to claim 1 further comprising at least two amplifying stages for amplifying said electrical signal(s) wherein the first stage is performed by first amplifying means being present in the detection unit (100), the second stage is performed by second amplifying means being present in the recording unit (200).

3. The device according to claim 2, wherein the ratio of the gain between the first amplifying means and the second amplifying means is at least of 5 and preferably superior to 10.

4. The device according to claims 2 or 3, wherein the first amplifying means are faster than the second amplifying means.

5. The device according to any of the claims 2 to 4, wherein the first amplifying means are generated more noise than the second amplifying means.

6. The device according to anyone of the preceding claims, wherein said detection unit (100) comprises amplification means arranged for amplifying said electrical signal.

7. The device according to anyone of the preceding claims, wherein said detection unit (100) comprises at least one comparator arranged to compare said amplified electrical signal to a predetermined threshold signal.

8. The device according to any of the preceding claims, wherein said recording unit (200) comprises amplification means.

9. A method for recording cell activity with a transducer or sensor (21) array comprising a plurality of transducers or sensors (21), wherein, for each transducer or sensor (21), the following steps are performed:
a) addressing a transducer or sensor (21) of the transducer array or addressing a transducer of a predetermined subset of transducers or sensors (21) of the transducer or sensor (21) array;
b) activating said transducer or sensor (21);
c) receiving from said activated transducer or sensor (21) any electrical signal possibly resulting from cell activity;
d) amplifying said electrical signal;
e) comparing said electrical signal to a predetermined electrical threshold signal;
f) determining by using the comparison result of step e) that said electrical signal corresponds to a cell activity;
g) for any electrical signal corresponding to a cell activity routing a recording channel to said activated transducer or sensor (21);
h) recording said electrical signal from said activated transducer or sensor (21)
**characterised in that** said method is performed in real-time.

10. The method according to claim 9, wherein the amplifying step comprises at least two stages performed with a first amplifier stage having a high gain, in the detection stage defined by steps a) to f), followed by a second amplifying stage at a low gain performed during the recording stage defined by steps g) to h).

11. The method according to claim 9 or 10 wherein the sequence of steps a) to h) for the next or subsequent transducer or sensor (21) starts between steps c) and d).

12. The method according to claim 9 or 10, wherein the steps a) to h) are repeated for the next or subsequent transducer or sensor (21).

13. The method according to any of the claims 9 to 12, further comprising - after step h) - the step of sending sequentially the recorded electrical signals to a post-processing unit.

14. The method according to any of the claims 9 to 13, wherein the step of determining - if the acquired electrical signal corresponds to a cell activity - is performed by a control unit (300).

15. The method according to any of the claims 9 to 14, wherein the ratio of the gain between the first amplifying stages and the second amplifying stages are at least of 5 and preferably superior to 10.

## Patentansprüche

1. Active-Interface-Vorrichtung, die umfasst:
- eine Anordnung von Messwandlern oder Sensoren (21), die eine Mehrzahl von Messwandlern oder Sensoren (21) umfasst, die so ausgelegt sind, dass sie eine Zellaktivität in ein elektrisches Signal umwandeln;
- zumindest eine Detektionseinheit (100) zum Detektieren des einen oder der mehreren elektrischen Signale;
- zumindest eine Aufzeichnungseinheit (200) zum Aufzeichnen des einen oder der mehreren elektrischen Signale, die eine Mehrzahl von Aufzeichnungskanälen umfasst, die so ausgelegt sind, dass sie zu den Messwandlern oder Sensoren (21) geleitet werden;
- zumindest eine Steuereinheit (300);
wobei die Steuereinheit (300) so ausgelegt ist, dass sie die Messwandler oder Sensoren (21) an die eine oder die mehreren Detektionseinheiten (100) adressiert, dass sie Messwandler oder Sensoren (21) aktiviert, und dass sie entscheidet, die Aufzeichnungskanäle bei einer Echtzeitdetektion von Zellaktivität durch die aktivierten Messwandler oder Sensoren zu jenen aktivierten Messwandlern oder Sensoren zu leiten, die die Zellaktivität detektiert haben.

2. Vorrichtung nach Anspruch 1 die darüber hinaus zumindest zwei Verstärkungsstufen zum Verstärken des einen oder der mehreren elektrischen Signale umfasst, wobei die erste Stufe mit einem ersten Verstärkungsmittel durchgeführt wird, das in der Detektionseinheit (100) vorhanden ist, wobei die zweite Stufe mit einem zweiten Verstärkungsmittel durchgeführt wird, das in der Aufzeichnungseinheit (200) vorhanden ist.

3. Vorrichtung nach Anspruch 2, wobei das Verstärkungsverhältnis zwischen dem ersten Verstärkungsmittel und dem zweiten Verstärkungsmittel zumindest 5 beträgt und vorzugsweise höher als 10 ist.

4. Vorrichtung nach Anspruch 2 oder 3, wobei das erste Verstärkungsmittel schneller als das zweite Verstärkungsmittel ist.

5. Vorrichtung nach einem der Ansprüche 2 bis 4, wobei das erste Verstärkungsmittel mehr Rauschen als das zweite Verstärkungsmittel erzeugt.

6. Vorrichtung nach einem der vorstehenden Ansprüche, wobei die Detektionseinheit (100) ein Verstärkungsmittel umfasst, das so ausgelegt ist, dass es das elektrische Signal verstärkt.

7. Vorrichtung nach einem der vorstehenden Ansprüche, wobei die Detektionseinheit (100) zumindest einen Komparator umfasst, der so ausgelegt ist, dass er das verstärkte elektrische Signal mit einem vorab festgelegten Grenzwertsignal vergleicht.

8. Vorrichtung nach einem der vorstehenden Ansprüche, wobei die Aufzeichnungseinheit (200) ein Verstärkungsmittel umfasst.

9. Verfahren zum Aufzeichnen von Zellaktivität mit einer Anordnung von Messwandlern oder Sensoren (21), die eine Mehrzahl von Messwandlern oder Sensoren (21) umfasst, wobei für jeden der Messwandler oder Sensoren (21) die folgenden Schritte durchgeführt werden:
a) Adressieren eines Messwandlers oder Sensors (21) der Messwandleranordnung oder Adressieren eines Messwandlers eines vorab festgelegten Teilsatzes von Messwandlern und Sensoren (21) der Anordnung von Messwandlern und Sensoren (21);
b) Aktivieren des Messwandlers oder Sensors (21);
c) Empfangen jedes elektrischen Signals vom aktivierten Messwandler oder Sensor (21), das womöglich aus einer Zellaktivität stammt;
d) Verstärken des elektrischen Signals;
e) Vergleichen des elektrischen Signals mit einem vorab festgelegten elektrischen Grenzwertsignal;
f) Ermitteln anhand des Vergleichsergebnisses von Schritt e), ob das elektrische Signal einer Zellaktivität entspricht;
g) in Bezug auf jedes elektrische Signal, das einer Zellaktivität entspricht, Leiten eines Aufzeichnungskanals zu dem aktivierten Messwandler oder Sensor (21);
h) Aufzeichnen des elektrischen Signals aus dem aktivierten Messwandler oder Sensor (21)
**dadurch gekennzeichnet, dass** das Verfahren in Echtzeit durchgeführt wird.

10. Verfahren nach Anspruch 9, wobei der Schritt des Verstärkens zumindest zwei Stufen umfasst, die mit einer ersten Verstärkerstufe mit einer hohen Verstärkung in der von den Schritten a) bis f) definierten Detektionsstufe durchgeführt werden, gefolgt von einer zweiten Verstärkungsstufe mit einer niedrigen Verstärkung, die während der durch die Schritte g) bis h) definierten Aufzeichnungsstufe durchgeführt wird.

11. Verfahren nach Anspruch 9 oder 10 wobei die Folge der Schritte a) bis h) für den nächsten oder darauf folgenden Messwandler oder Sensor (21) zwischen den Schritten c) und d) beginnt.

12. Verfahren nach Anspruch 9 oder 10, wobei die Schritte a) bis h) für den nächsten oder darauf folgenden Messwandler oder Sensor (21) wiederholt werden.

13. Verfahren nach einem der Ansprüche 9 bis 12, das nach Schritt h) darüber hinaus den Schritt des sequentiellen Sendens der aufgezeichneten elektrischen Signale an eine Nachverarbeitungseinheit umfasst.

14. Verfahren nach einem der Ansprüche 9 bis 13, wobei der Schritt des Ermittelns, ob das erfasste elektrische Signal einer Zellaktivität entspricht, mit einer Steuereinheit (300) durchgeführt wird.

15. Verfahren nach einem der Ansprüche 9 bis 14, wobei das Verstärkungsverhältnis zwischen den ersten Verstärkungsstufen und den zweiten Verstärkungsstufen zumindest 5 beträgt und vorzugsweise höher als 10 ist.

## Revendications

1. Dispositif d'interface active comprenant :
un réseau de transducteurs ou de capteurs (21) comprenant une pluralité de transducteurs ou de capteurs (21) conçus pour transformer une activité cellulaire en un signal électrique ;
au moins une unité de détection (100) pour détecter le(s) signal(aux) électrique(s) ;
au moins une unité d'enregistrement (200) pour enregistrer le(s) signal(aux) électrique(s), comprenant une pluralité de canaux d'enregistrement conçus pour être routés vers lesdits transducteurs ou capteurs (21) ;
au moins une unité de commande (300) ;
où ladite unité de commande (300) est conçue pour associer lesdits transducteurs ou capteurs (21) à(aux) l'unité(s) de détection (100), pour activer les transducteurs ou capteurs (21), et pour décider par détection en temps réel d'une activité cellulaire via lesdits transducteurs ou capteurs activés de router en temps réel les canaux d'enregistrement vers ces transducteurs ou capteurs activés qui ont détecté une activité cellulaire.

2. Dispositif selon la revendication 1, comprenant en outre au moins deux phases d'amplification pour amplifier le(s)dit(s) signal(aux) électrique(s) où la première phase est réalisée par des premiers moyens d'amplification présents dans l'unité de détection (100) et la deuxième phase réalisée par des deuxièmes moyens d'amplification présents dans l'unité d'enregistrement (200).

3. Dispositif selon la revendication 2, dans lequel le rapport de gain entre les premiers moyens d'amplification et les seconds moyens d'amplification est au moins de 5, et de préférence supérieur à 10.

4. Dispositif selon la revendication 2 ou 3, dans lequel les premiers moyens d'amplification sont plus rapides que les deuxièmes moyens d'amplification.

5. Dispositif selon l'une quelconque des revendications 2 à 4, dans lequel les premiers moyens d'amplification génèrent plus de bruit que les deuxièmes moyens d'amplification.

6. Dispositif selon l'une quelconque des revendications précédentes, dans lequel ladite unité de détection (100) comprend des moyens d'amplification conçus pour amplifier ledit signal électrique.

7. Dispositif selon l'une quelconque des revendications précédentes, dans lequel ladite unité de détection (100) comprend au moins un comparateur conçu pour comparer ledit signal électrique amplifié à un signal seuil prédéterminé.

8. Dispositif selon l'une quelconque des revendications précédentes, dans lequel ladite unité d'enregistrement (200) comprend des moyens d'amplification.

9. Procédé pour enregistrer une activité cellulaire avec un réseau de transducteurs ou de capteurs (21) comprenant une pluralité de transducteurs ou de capteurs (21), où, pour chaque transducteur ou capteur (21), les étapes suivantes sont exécutées :
a) solliciter un transducteur ou un capteur (21) du réseau de transducteurs ou solliciter un transducteur d'un sous-ensemble prédéterminé de transducteurs ou de capteurs (21) du réseau de transducteurs ou de capteurs (21) ;
b) activer ledit transducteur ou capteur (21) ;
c) recevoir dudit transducteur ou capteur (21) activé tout signal électrique pouvant résulter d'une activité cellulaire ;
d) amplifier ledit signal électrique ;
e) comparer ledit signal électrique à un signal électrique seuil prédéterminé ;
f) déterminer, au moyen du résultat comparatif de l'étape e), si ledit signal électrique correspond à une activité cellulaire ;
g) pour tout signal électrique correspondant à une activité cellulaire, router un canal d'enregistrement vers ledit transducteur ou capteur (21) activé ;
h) enregistrer ledit signal électrique provenant dudit transducteur ou capteur (21) activé,
**caractérisé en ce que** ledit procédé est exécuté en temps réel.

10. Procédé selon la revendication 9, dans lequel l'étape d'amplification comprend au moins deux phases, une première phase d'amplification avec un gain élevé étant réalisée lors de la phase de détection définie par les étapes a) à f), suivie par une deuxième phase d'amplification avec un faible gain réalisée lors de la phase d'enregistrement définie par les étapes g) à h).

11. Procédé selon la revendication 9 ou 10, dans lequel la séquence des étapes a) à h) pour le prochain transducteur ou capteur (21) ou le suivant commence entre les étapes c) et d).

12. Procédé selon la revendication 9 ou 10, dans lequel les étapes a) à h) sont répétées pour le prochain transducteur ou capteur (21) ou le suivant.

13. Procédé selon l'une quelconque des revendications 9 à 12, comprenant en outre, après l'étape h), l'étape consistant à envoyer de façon séquentielle les signaux électriques enregistrés à une unité de post-traitement.

14. Procédé selon l'une quelconque des revendications 9 à 13, dans lequel l'étape consistant à déterminer si le signal électrique acquis correspond à une activité cellulaire est réalisée par une unité de commande (300).

15. Procédé selon l'une quelconque des revendications 9 à 14, dans lequel le rapport de gain entre les premières phases d'amplification et les deuxièmes phases d'amplification est au moins de 5, et de préférence supérieur à 10.
